Europäisches Patentamt

European Patent Office

Office européen des brevets

(11) Publication number: **0 338 679**
**A2**

## EUROPEAN PATENT APPLICATION

(21) Application number: 89302712.8

(22) Date of filing: 20.03.89

(51) Int. Cl.⁴: **A61K 37/02** , **A61K 37/24** ,
**A61K 45/02**

(30) Priority: 24.03.88 US 172728

(43) Date of publication of application:
25.10.89 Bulletin 89/43

(84) Designated Contracting States:
AT BE CH DE ES FR GB GR IT LI LU NL SE

(71) Applicant: GENENTECH, INC.
460 Point San Bruno Boulevard
South San Francisco California 94080(US)

(72) Inventor: Sherwin, Stephen A.
3311 Washington Street
San Francisco California 94118(US)

(74) Representative: Armitage, Ian Michael et al
MEWBURN ELLIS & CO. 2/3 Cursitor Street
London EC4A 1BQ(GB)

(54) Tumour necrosis factor in the treatment of bladder cancer.

(57) The use of TNF to treat bladder tumors is un-
expectedly enhanced by administering the TNF by
intravesical infusion.

EP 0 338 679 A2

## TUMOUR NECROSIS FACTOR IN THE TREATMENT OF BLADDER CANCER

This invention relates to the therapy of bladder cancer. In particular, it relates to the use of tumor necrosis factors in the treatment of bladder cancer.

Tumor necrosis factors are polypeptides having cytolytic or cytotoxic activity on tumor cells. At present there are two forms of tumor necrosis factor (hereinafter, collectively or individually "TNF"). TNF-$\alpha$ and TNF-$\beta$, together with nucleic acid encoding each and recombinant methods for their synthesis, are respectively described in Pennica et al., Nature 312:724 (1984) and TNF-$\beta$ Gray et al., Nature 312:721 (1984). Numerous amino acid sequence, glycosylation and other variants for each molecule have been described and are included within the scope of the term "TNF". For example, see EP 148,311; 90,892; 132,125; 221,321; 205,038; 250,000; 251,037; 155,549; 220,966; 232,107; 168,214; and 164,965; and WO 86/03751. T24 bladder tumor cells have been reported to be refractory in vitro to the cytotoxic or cytostatic effect of TNF, notwithstanding tumors originating in other organs are susceptible to TNF cytotoxicity or cytostasis. Limited data now available suggests that intravenous administration of TNF has been unsuccessful in achieving a favorable response in bladder cancers, and to date systemically administered TNF has not shown the extensive in vivo therapeutic value initially suggested by in vitro studies. In addition, TNF has a brief biological half-life and produces undesirable side effects when used in high doses.

The literature contains numerous suggestions, most of them apparently hypothetical, for the administration of TNF in the therapy of neoplasms. For example, EP 90,892 teaches parental or topical administration; EP 100,641 oral, parenteral or topical; EP 132,125 rectal, oral, parenteral or topical; EP 221,321 intravenous, oral, intraarterial, subcutaneous, intraepidermal, intralymphatic, "intracorporeal" or rectal; EP 155,549 by injection, eye drop, nasal drop, inhalation, "external preparation", oral, rectal, vaginal, intramuscular or intravenous routes; EP 164,965 and EP 168,214, by intravenous, intraperitoneal, subcutaneous, intramuscular, or intralesional injection or infusion of TNF-$\alpha$ or TNF-$\beta$, as such or in sustained release formulations and WO 86/03751 by subcutaneous, intravenous, intramuscular or intralesional administration. These extensive disclosures are devoid of any teaching or suggestion to employ intravesical administration.

Intravesical administration of an agent comprises catheterizing the patient's urinary tract and infusing a solution or other preparation into the bladder. This procedure per se is widely known to practitioners. It has been used in the treatment of bladder cancers as a convenient and effective mechanism for delivering therapeutic agents to the site of the lesion. See, for example, Messing et al. in Zing et al., Ed. Bladder Cancer, pp. 235-262 (1985); Orihuela et al., Cancer 60:326-333 (1987); Droller et al., Cancer 60:635-644 (1987); and Richie et al. in DeVita et al., Cancer, Principles and Practice of Oncology, 2nd edition, pp. 915-928 (1985). Practical experience in the therapy of bladder cancer has demonstrated that many of these tumors are resistant to conventional chemotherapeutic agents.

The present invention may provide an effective therapy for patients suffering from bladder cancer; a means of preventing the recurrence of bladder cancer after an initial course of therapy; a minimization of the side effects of TNF therapy in the treatment of bladder cancer; and an increase in the therapeutic efficacy of TNF in vivo.

This invention is directed to means enabling the treatment of a bladder tumor, or its prophylaxis, by the intravesical administration of a therapeutically effective dose of TNF. Surprisingly, this route of administration was found to result in less TNF toxicity. TNF was found to not be absorbed into the blood stream through the bladder mucosa, at least in amounts sufficient to produce significant systemic toxicity. Also surprising was that bladder cancers were found to be responsive to TNF by this route of administration, notwithstanding that bladder tumors were in general not affected by TNF in in vitro studies or by intravenous administration and that the tumors treated were in many cases previously found to be refractory to other conventional therapies for bladder cancer.

### Detailed Description of the Invention

The bladder tumors to be treated by the method of this invention are either benign or neoplastic (papillomas and transitional cell carcinomas I, II or III), superficial or invasive. It is not necessary that the presence of such tumors be confirmed. The TNF may be used after transurethral resection of tumors or in prophylaxis of tumors that are not visible to cytoscopic inspection and for which a patient is simply believed to be at risk, e.g. as a result of exposure to a carcinogen.

TNF as defined herein means TNF-$\alpha$ and TNF-$\beta$, together with their amino acid, glycosylation and other variants or derivatives. The literature discussed supra describes TNF-$\alpha$ and TNF-$\beta$, as well

as many of their variants. It is expected that other variants and derivatives will become available in the future, and these are to be considered to fall within the scope of this invention. The TNF may be obtained by recombinant synthesis (rTNF) or by purification from nonrecombinant sources such as cultures of induced lymphoblastoid or monocytic cell lines. TNF from other animal species can be used in the treatment of human tumors, and vice versa, although it is preferable to use TNF from the same species being treated.

The therapeutically effective dosage of TNF to be employed by intravesical administration generally will range about from 1 mcg/m$^2$ to 500 mcg/m$^2$, and is ordinarily about from 5 mcg/m$^2$ to 50 mcg/m$^2$. This dosage has been found to be appropriate for TNF-$\alpha$ having the native mature amino acid sequence described by Pennica et al. supra, and which is at least >95% pure by weight of protein. It will be appreciated that the practitioner will adjust the therapeutic dose in line with clinical experience for any given TNF. The efficacy of the dosage will be followed in the same fashion as heretofore employed chemotherapeutic agents. If the response is inadequate then the dosage will be increased until unacceptable toxicity is encountered. The side-effects of TNF previously experienced by patients receiving intravenous therapy include fever and shaking rigors (chills) and, rarely, severe hypotension. Obviously, the clinician will need to balance the adverse effects of TNF administration against the therapeutic objectives. However, this threshold has not yet been reached with intravesical TNF therapy.

TNF optionally is administered together with other agents or therapies heretofore employed in the intravesical therapy of bladder tumors. Therapies or agents which are used optionally in a course of therapy with intravesical TNF include, for example, alkylating agents such as melphalan, cyclophosphamide, ifosfamide, estramustine sodium phosphate, busulfan, imorposulfan tosilate, N-methyl-3,3′-dimesyloxydipropylamine biphenyl-4,4′-disulfonate, carboquone, thio-TEPA, carmustine, nimustine hydrochloride, streptozocin, dacarbazine, and pipobroman; metabolic antagonists such as methotrexate, fluorouracil, tegaful, carmoful, cytarabine, ancitabine hydrochloride, enocitabine, 6-mercaptopurine, and thioinosine; antioncotic antibiotics such as doxorubicin, daunorubicin, aclarubicin, bleomycin, peplomycin, mitomycin C, epodyl, tenopside, actinomycins D and C, chromomycin A$_3$, mithramycin, and neocarzinostatin; plant alkaloids such as vincristine sulfate, vinblastine sulfate, vindesine sulfate, and podophyllotoxin; BCG; oral pyridoxine; radiation; photodynamic therapy; interferon; interleukin 2; prostaglandin synthetase inhibitors; cis-platinum;

and hypothermia. These other agents or therapies are used at the same time as TNF is administered or in a sequential course of therapy.

TNF for intravesical administration typically is formulated into a solution, generally about 50 ml, which is isotonic and sterile. Saline is a suitable carrier, although other conventional parenteral solutions or buffers are usable. The formulation may contain agents such as nonionic detergents, albumin and the like previously suggested for use in TNF dosage forms. The TNF also is suitably encapsulated, for example in multilamellar or unilamellar liposomes, or formulated together with colloids or thickening agents suitable for passage through the urinary tract, in order to extend and enhance the therapeutic activity of the TNF.

Intravesical TNF administration desirably is used in the context of post-operative treatment, i.e., it is employed after surgical removal of tumors and polyps, or it is used on a prophylactic basis to treat tumors not evident to visual inspection or it is used when surgical therapy is no longer possible.

## Example 1

Patient #178001, D.A.D., is a 65 year old male with a long history of superficial bladder cancer. In the past, he has required multiple surgeries but has repeatedly recurred despite surgical resection. He has also been treated with intravesical administration of thiotepa and BCG. Despite the immunotherapy and chemotherapy, the patient had recurrent disease with massive replacement of the mucosa of the bladder by tumor prior to beginning rTNF therapy. The patient began rTNF administered into the bladder twice weekly for two hours. The instillations were done through a standard urinary catheter with albumin added to prevent adsorption to the plastic surface. The rTNF was retained in the bladder for 2 hours prior to voiding. The patient received a dose of 10 mcg/m$^2$ with each instillation. At the end of the 4 week treatment period, the patient was again cystoscoped and was felt to have stable disease. The patient was not treated for approximately one month, at which time he returned for repeat cystoscopy. He had had no intervening therapy since discontinuing rTNF. At that evaluation, he had more than a 50% reduction in visible tumor. The patient was restarted at his previous dose of 10 mcg/m$^2$ administered twice weekly.

## Example 2

Patient #178002, R.M.W., is a 33 year old male who had undergone multiple surgeries for recurrent superficial bladder cancer. He had also received BCG therapy but had had a documented recurrence. At the time of initial therapy, the patient had two lesions; one easily observable lesion was designated the marker lesion, since the second lesion was protruding from the ureter and could not be completely evaluated. After one month of therapy (10 mcg/m² twice weekly), the measurable lesion had completely disappeared and the amount of tumor protruding from the ureter was decreased. However, this lesion was not completely evaluable and the patient required a stint for ureteral obstruction. The patient was continued on therapy at his dose of 10 mcg/m² twice weekly.

Example 3

Patient #178003, C.R.S., is a 59 year old male who presented with recurrent superficial bladder cancer despite multiple previous surgeries and prior thioTEPA therapy. At the time of initial presentation, the patient was found to have six papillary tumors and he was started on rTNF, which was administered by urinary catheter twice weekly for four weeks at a dose of 25 mcg/m². The cystoscopy done after four weeks of therapy showed a complete resolution of the six papillary tumors. The patient was continued on maintenance therapy. No significant toxicity has been noted.

**Claims**

1. The use of tumour necrosis factor (TNF) for the preparation of a medicament for intravesical administration, for the therapeutic and/or prophylactic treatment of bladder tumour.

2. The use of TNF according to claim 1 wherein the medicament contains a therapeutically effective dose of 1 mcg/m² to 500 mcg/m² TNF.

3. The use of TNF according to claim 1 or claim 2 wherein the TNF is a TNF-α.

4. The use of TNF according to claim 1 or claim 2 wherein the TNF is a TNF-β.

5. The use of TNF according to any one of the preceding claims wherein the medicament includes an interferon.

6. The use of TNF according to claim 5 wherein the interferon is gamma interferon.

7. The use of TNF according to any one of the preceding claims wherein the TNF is encapsulated.

8. The use of TNF according to claim 7 wherein the TNF is present in a liposome.

9. The use of TNF according to any one of the preceding claims wherein medicament is for treatment of a patient thought to be at risk for bladder cancer but not yet diagnosed as having bladder cancer.

10. The use of TNF according to any one of the preceding claims wherein the TNF is for administering after resection of a bladder tumour.

11. The use of TNF according to any one of the preceding claims wherein the amount of TNF in the medicament would have been toxic had it been administered intravenously to the patient.